# EUROPEAN PATENT APPLICATION

(11) **EP 4 667 933 A1**
(43) Date of publication of application: **24.12.2025**
(21) Application number: 24756029.5
(22) Date of filing: 02.02.2024
(51) Int. Cl.: G01N 33/00

(54) **CARBON CAPTURE ABSORBENT PERFORMANCE TESTING SYSTEM**

(30) Priority: 17.02.2023 CN 202310132789
(71) Applicant: Huaneng Clean Energy Research Institute, Beijing 102209 (CN); Huaneng Longdong Energy Co., Ltd Zhengning Power Plant, Qingyang, Gansu 745306 (CN); Huaneng Group R&D Center Co., Ltd., Beijing 100031 (CN)
(72) Inventor: FAN, Zian, Beijing 102209 (CN); MI, Lihai, Beijing 102209 (CN); CAO, Zhiyang, Beijing 102209 (CN); LIU, Hanming, Beijing 102209 (CN); GONG, Peng, Beijing 102209 (CN); WANG, Huanjun, Beijing 102209 (CN); NIU, Hongwei, Beijing 102209 (CN); LIU, Lianbo, Beijing 102209 (CN); GUO, Dongfang, Beijing 102209 (CN)
(74) Representative: advotec.
(86) International application number: PCT/CN2024/075473
(87) International publication number: WO 2024/169655

(57) **Abstract**

A carbon capture absorbent performance testing system. The carbon capture absorbent performance testing system comprises a carbon capture unit (1), an outer circulation unit (2) and a measurement assembly (3). The carbon capture unit (1) can allow carbon dioxide to react with an absorbent solution. The outer circulation unit (2) comprises an outer circulation pipeline (23), a temperature adjustment device (22), and a driving device (21). The temperature adjustment device (22) and the driving device (21) are both arranged on the outer circulation pipeline (23), the temperature adjustment device (22) can adjust and control the temperature of the absorbent solution in the outer circulation pipeline (23), and the driving device (21) can drive the absorbent solution to circularly flow between the outer circulation pipeline (23) and the carbon capture unit (1). The measurement assembly (3) can measure the carbon capture rate of the carbon capture unit (1).

## Description

### CROSS-REFERENCE TO RELATED APPLICATION

This application claims priority to and benefits of Chinese Patent Application Serial No. 202310132789.5, filed with the China National Intellectual Property Administration on February 17, 2023, the entire content of which is incorporated herein by reference.

### FIELD

The present disclosure relates to a field of carbon capture technologies, and particularly to a carbon capture absorbent performance testing system.

### BACKGROUND

At present, an industrial carbon capture technical means mainly includes a chemical absorption method and a physical adsorption method, the chemical absorption method mainly utilizes an absorbent solution to separate carbon dioxide from industrial flue gas for collection, and the reaction principle is as follows: after the reaction between the absorbent solution and the carbon dioxide, a kind of steady-state compound solution is formed, and then the compound solution is re-decomposed and restored to carbon dioxide gas and the absorbent solution under the action of high temperature, thus realizing separate collection of carbon dioxide, and also recycling the absorbent solution for reuse.

In the related art, under the influence of temperature changes in various processes of carbon capture, such as a heat exchange process of a lean solution and a rich solution, a heating process of a desorption tower, a reflux cooling process, etc., the absorbent is prone to volatilization or degradation, and a certain degree of absorbent loss exists. Therefore, it is necessary to replenish the absorbent in the process of carbon capture in order to maintain the normal progress of the carbon capture. However, it is difficult to specify a specific loss rate of the absorbent in various processes of carbon capture, resulting in that an operator is unable to replenish the absorbent as required, and thus affecting the carbon capture efficiency.

### SUMMARY

Embodiments of the present disclosure provide a carbon capture absorbent performance testing system, the carbon capture absorbent performance testing system may simulate various processes in a carbon capture industrial circulation system, simulate temperatures of various processes in an actual working condition by a temperature adjustment device, and derive a loss amount of the absorbent in an actual carbon capture process by detecting a loss amount of a content of the absorbent in a simulated working condition, so as to provide a basis for reasonably replenishing an usage amount of the absorbent. In addition, the carbon capture absorbent performance testing system of the embodiment of the present disclosure may also be used to observe an affecting factor (such as a temperature of a absorption tower, a heating temperature of a desorption tower, and a material for manufacturing a carbon capture device) of a loss velocity of the absorbent, so as to be able to reduce the loss rate of the absorbent by an experiment result and optimize the carbon capture process.

The carbon capture absorbent performance testing system of the embodiment of the present disclosure includes: a carbon capture unit configured to make carbon dioxide react with an absorbent solution to absorb the carbon dioxide; an external circulation unit including an external circulation pipeline, a temperature adjustment device, and a driving device, in which an inlet of the external circulation pipeline is communicated with a liquid outlet of the carbon capture unit, and an outlet of the external circulation pipeline is communicated with a liquid inlet of the carbon capture unit, the temperature adjustment device and the driving device are arranged in the external circulation pipeline, the temperature adjustment device is configured to adjust and control a temperature of the absorbent solution in the external circulation pipeline, and the driving device is configured to drive the absorbent solution to circularly flow between the external circulation pipeline and the carbon capture unit; and a detection assembly configured to detect a carbon capture rate of the carbon capture unit.

In the carbon capture absorbent performance testing system according to the embodiment of the present disclosure, the external circulation unit may utilize the driving device to transfer the absorbent solution from the carbon capture unit to the temperature adjustment device, then the temperature adjustment device may simulate temperature changes of the absorbent solution in the actual carbon capture process by adjusting the temperature of the absorbent solution, then the absorbent solution after temperature adjustment returns to the carbon capture unit from the external circulation pipeline and reacts with the carbon dioxide, and finally the detection assembly may detect the carbon capture rates of the absorbent solutions with and without temperature adjustment, so that two sets of data may be compared to derive a change in the carbon dioxide absorption capacity of the absorbent solution under the simulated working condition, thus deriving the loss rate of the absorbent. Therefore, the carbon capture absorbent performance testing system of the embodiment of the present disclosure simulates various processes of the actual carbon capture industrial circulation by a simplified experimental equipment, simulates the temperatures of various processes in an actual working condition by the temperature adjustment device, and derives the loss amount of the absorbent in the actual carbon capture process by detecting the loss amount of the content of the absorbent in the simulated working condition, thereby providing a basis for reasonably replenishing the usage amount of the absorbent, and maintaining a stable carbon capture efficiency.

In addition, the carbon capture absorbent performance testing system of the embodiment of the present disclosure may also be used to observe the affecting factor (such as the temperature of the absorption tower, the heating temperature of the desorption tower, the material for manufacturing the carbon capture device, etc.) of the loss velocity of the absorbent, so as to reduce the loss rate of the absorbent by experimental results and optimize the carbon capture process.

In some embodiments, the detection assembly includes a first detection member and a second detection member, the first detection member is arranged at a gas inlet of the carbon capture unit to detect a content of carbon dioxide in incoming gas, and the second detection member is arranged at a gas outlet of the carbon capture unit to detect a content of carbon dioxide in outgoing gas.

In some embodiments, the temperature adjustment device includes a heater and a heat exchanger, and the external circulation pipeline includes a liquid intake pipeline in communication with the liquid outlet of the carbon capture unit and a liquid return pipeline in communication with the liquid inlet of the carbon capture unit. A cold source chamber of the heat exchanger is communicated with the liquid intake pipeline, and a heat source chamber of the heat exchanger is communicated with the liquid return pipeline. The heater is arranged in a pipeline between an outlet of the cold source chamber and an inlet of the heat source chamber.

In some embodiments, the temperature adjustment device further includes a cooler, and the cooler is arranged in an outlet pipeline of the heat source chamber to cool a high temperature absorbent solution flowing out of the heat source chamber.

In some embodiments, the heater is an electric heater or a heat pipe heater.

In some embodiments, the external circulation unit further includes a defoamer, the driving device is arranged between the liquid outlet of the carbon capture unit and the temperature adjustment device, and the defoamer is arranged between the driving device and the temperature adjustment device.

In some embodiments, the external circulation unit further includes a metering pump, and the metering pump is arranged between the defoamer and the temperature adjustment device.

In some embodiments, the external circulation unit further includes a back pressure valve, and the back pressure valve is arranged in the external circulation pipeline.

In some embodiments, the carbon capture absorbent performance testing system further includes a temperature detection assembly, the temperature detection assembly may be configured to detect temperatures at the liquid inlet and the liquid outlet of the carbon capture unit, and temperatures at an inlet and an outlet of each element of the external circulation unit.

In some embodiments, the temperature detection assembly includes a plurality of thermometers, the plurality of the thermometers are correspondingly arranged at respective temperature measurement points, and the plurality of thermometers are K-type stainless steel thermocouples.

### BRIEF DESCRIPTION OF THE DRAWINGS

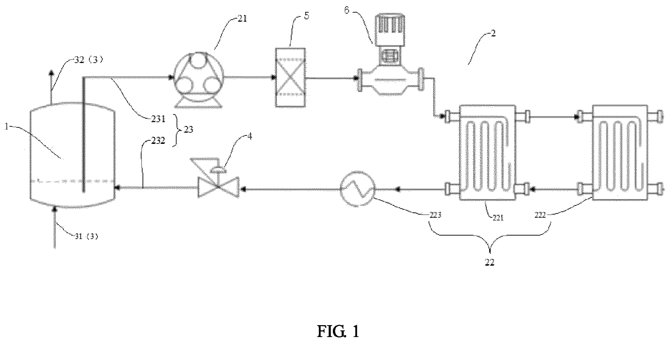

Fig. 1 is a schematic diagram of a carbon capture absorbent performance testing system according to an embodiment of the present disclosure.

### Reference signs:

carbon capture unit 1, external circulation unit 2, driving device 21, temperature adjustment device 22, heat exchanger 221, heater 222, cooler 223, external circulation pipeline 23, liquid intake pipeline 231, liquid return pipeline 232, detection assembly 3, first detection member 31, second detection member 32, back pressure valve 4, defoamer 5, metering pump 6.

### DETAILED DESCRIPTION

Embodiments of the present disclosure are described in detail below, and examples of the embodiments are shown in the accompanying drawing. The embodiments described below by reference to the accompanying drawing are illustrative and are intended to be used to explain the present disclosure and are not to be construed as a limitation of the present disclosure.

As shown in FIG. 1, a carbon capture absorbent performance testing system of an embodiment of the present disclosure includes a carbon capture unit 1, an external circulation unit 2, and a detection assembly 3.

Specifically, the carbon capture unit 1 is configured to make carbon dioxide react with an absorbent solution so as to absorb carbon dioxide. The external circulation unit 2 includes an external circulation pipeline 23, a temperature adjustment device 22, and a driving device 21. An inlet of the external circulation pipeline 23 is communicated with a liquid outlet of the carbon capture unit 1, and an outlet of the external circulation pipeline 23 is communicated with a liquid inlet of the carbon capture unit 1. The temperature adjustment device 22 and the driving device 21 are both arranged in the external circulation pipeline 23, and the temperature adjustment device 22 is configured to adjust and control a temperature of the absorbent solution within the external circulation pipeline 23. The driving device 21 is configured to drive the absorbent solution to circularly flow between the external circulation pipeline 23 and the carbon capture unit 1. The detection assembly 3 is configured to detect a carbon capture rate of the carbon capture unit 1.

It may be noted that the carbon capture absorbent performance testing system of the embodiment of the present disclosure is designed based on the temperature adjustment process undergone by the absorbent solution in the actual production. That is, in the actual production, the absorbent solution firstly absorbs carbon dioxide in industrial flue gas in an absorption tower to form a carbon dioxide rich solution, then the carbon dioxide rich solution flows into the desorption tower to be desorbed at a high temperature, and the carbon dioxide rich solution is decomposed into carbon dioxide gas and a carbon dioxide lean solution (i.e., the original absorbent solution), and then the carbon dioxide gas enters a gas storage tank for storage, and the carbon dioxide lean solution after cooling flows into the absorption tower again to participate in the next carbon capture circulation. In this process, a high temperature carbon dioxide rich solution also exchanges heat with a low temperature carbon dioxide rich solution to enhance the utilization of residual heat in the carbon capture process.

That is to say, in the carbon capture absorbent performance testing system of the embodiment of the present disclosure, the carbon capture unit 1 may replace the absorption tower to enable the absorbent solution to absorb the carbon dioxide, the external circulation unit 2 may utilize the driving device 21 to transfer the absorbent solution from the carbon capture unit 1 to the temperature adjustment device 22, and then the temperature adjustment device 22 may simulate the temperature changes of the absorbent solution, which occur in carbon capture processes such as heating in the desorption tower, heat exchange of the rich solution and the lean solution, and recovery and cooling of the lean solution, etc., by adjusting the temperature of the absorbent solution. Then, the absorbent solution after temperature adjustment returns to the carbon capture unit 1 from the external circulation pipeline 23 and reacts with the carbon dioxide, and finally the detection assembly 3 may detect the change of the carbon dioxide content in the carbon capture unit 1 to reflect the carbon capture rate of the absorbent solution.

In order to facilitate the understanding of a method of using the carbon capture absorbent performance testing system of the embodiment of the present disclosure, the method of using the carbon capture absorbent performance testing system is now explained in conjunction with a carbon capture absorbent performance testing process. In some embodiments, when conducting an experiment, a set of absorbent solution may be prepared to directly conduct carbon capture in the carbon capture unit 1 (i.e., at this time, the external circulation unit 2 is closed, and the temperature of the absorbent solution is not adjusted), and the detection assembly 3 records the carbon capture rate of this set of absorbent solution. Also, another set of absorbent solution with the same concentration as the above set of absorbent solution is prepared, and the temperature of the other set of absorbent solution is adjusted by the external circulation unit 2. Then, the other set of absorbent solution carries out the carbon capture in the carbon capture unit 1, and the detection assembly 3 also records the carbon capture rate of this set of absorbent solution. Finally, the carbon capture rates of the two sets of absorbent solution may be compared to derive a loss rate of the absorbent under the simulated working condition.

It may be understood that the loss of the absorbent is reflected as a decrease of an absorbent concentration in the solution, and the absorbent concentration is difficult to measure accurately. Therefore, in the embodiment of the present disclosure, the change of the carbon capture rate of the absorbent solution before and after the temperature adjustment may be utilized to indirectly indicate the loss rate of the absorbent, i.e., the higher the carbon capture rate of the absorbent solution is, the lower the loss rate of the absorbent is, and conversely, the lower the carbon capture rate of the absorbent solution is, the lower the loss rate of the absorbent is.

It may be understood that when the same kind of absorbent solution participates in the carbon capture processes under different temperature regulation modes, the loss rate of the absorbent is different, or when different kinds of absorbent solutions participate in the carbon capture process under the same regulation mode, the loss rate of the absorbent is different. However, the embodiment of the present disclosure simulates the temperature change undergone by the absorbent solution in the actual carbon capture process by a simplified experimental equipment, so that not only the temperature of the absorbent solution may be precisely adjusted in accordance with the actual working condition, but also the influence of other external factors on the experimental results may be avoid, so as to derive the accurate loss rate of the absorbent under various working conditions, thereby providing a basis for reasonably replenishing the usage amount of the absorbent during the actual carbon capture process, and maintaining a stable carbon capture efficiency.

It may be understood that the external circulation unit 2 leads out the absorbent solution in the carbon capture unit 1 for temperature adjustment and control, which is more convenient for realizing a temperature regulation mode consistent with the actual working condition, thereby enhancing the accuracy of the experiment.

In some embodiments, the driving device 21 may adopt a peristaltic pump, and the peristaltic pump may realize the delivery of liquid by squeezing a hose, and thus the absorbent solution in the pump only passes through the hose, and is not prone to leaving residues, so as to achieve extremely easy cleaning, and realize non-polluting delivery, thus ensuring that the carbon capture absorbent performance testing system provided by the embodiment of the present disclosure may be used multiple times, and enhancing the experimental efficiency.

In addition, the carbon capture absorbent performance testing system of the embodiment of the present disclosure may also be used to observe affecting factors of a loss velocity of the absorbent. In some embodiments, a change situation of the carbon capture rate of the absorbent solution may be observed by changing a temperature of an initial absorbent solution in the carbon capture unit 1, thereby deriving the effect of the temperature of the absorption tower on the loss velocity of the absorbent. In some embodiments, the change situation of the carbon capture rate of the absorbent solution may also be observed by changing a maximum heating temperature in the temperature adjustment device 22, thereby deriving the effect of the heating temperature of the desorption tower on the loss velocity of the absorbent. In some embodiments, the change situation of the carbon capture rate of the absorbent solution may also be observed by changing materials for manufacturing various assemblies of the carbon capture absorbent performance testing system, thereby deriving the effect of the materials through which the absorbent solution passes on the loss velocity of the absorbent. Thus, the loss rate of the absorbent may be reduced by the experimental results and the carbon capture process may be optimized.

The carbon capture absorbent performance testing system according to the embodiment of the present disclosure, the external circulation unit may utilize the driving device to transfer the absorbent solution from the carbon capture unit to the temperature adjustment device, then the temperature adjustment device may simulate the temperature change of the absorbent solution in the actual carbon capture process by adjusting the temperature of the absorbent solution, then the absorbent solution after temperature adjustment may return to the carbon capture unit from the external circulation pipeline and react with the carbon dioxide, and finally, the detection assembly may detect the carbon capture rates of the absorbent solutions with and without temperature adjustment, so that two sets of data may be compared to derive the change of the carbon dioxide absorption capacity of the absorbent solution under the simulated working condition, thus deriving the loss rate of the absorbent. Therefore, the carbon capture absorbent performance testing system of the embodiment of the present disclosure simulates various processes in the actual carbon capture industrial circulation by the simplified experimental equipment, simulates the temperatures of various processes in the actual working condition by the temperature adjustment device, and derives the loss amount of the absorbent in the actual carbon capture process by detecting a loss amount of a content of the absorbent in the simulated working condition, so as to provide a basis for reasonably replenishing the usage amount of the absorbent and maintain a stable carbon capture efficiency.

In addition, the carbon capture absorbent performance testing system of the embodiment of the present disclosure may also be used to observe the affecting factors of the loss velocity of the absorbent, such as the temperature of the absorption tower, the heating temperature of the desorption tower, the material for manufacturing the carbon capture device, etc., so as to reduce the loss rate of the absorbent by the experiment results and optimize the carbon capture process.

In some embodiments, as shown in FIG. 1, the detection assembly 3 includes a first detection member 31 and a second detection member 32, the first detection member 31 is arranged at a gas inlet of the carbon capture unit 1 for detecting a content of carbon dioxide in incoming gas, and the second detection member 32 is arranged at a gas outlet of the carbon capture unit 1 for detecting a content of carbon dioxide in outgoing gas.

It may be understood that the carbon capture rate of the absorbent solution is expressed as the content of carbon dioxide absorbed by the absorbent solution. In the embodiment of the present disclosure, after mixed gas containing carbon dioxide is introduced into the carbon capture unit 1, the first detection member 31 is used to detect the content of carbon dioxide in the mixed gases before reacting with the absorbent solution, and the second detection member 32 is used to detect the content of carbon dioxide in the mixed gas after reacting with the absorbent solution. The reduced amount of carbon dioxide may be derived by comparing the two sets of detection data, and the reduced amount of carbon dioxide is the amount of carbon dioxide absorbed by the absorbent solution, thereby determining the carbon capture rate of the absorbent solution.

It may be understood that the detection assembly 3 may detect the change in the content of carbon dioxide in a variety of ways, such as a carbon isotope labeling method, an infrared absorption method, etc., which may be reasonably selected according to the experimental condition.

In some embodiments, as shown in FIG. 1, the temperature adjustment device 22 includes a heater 222 and a heat exchanger 221, the external circulation pipeline 23 includes a liquid intake pipeline 231 in communication with a liquid outlet of the carbon capture unit 1 and a liquid return pipeline 232 in communication with a liquid inlet of the carbon capture device, a cold source chamber of the heat exchanger 221 is communicated with the liquid intake pipeline 231, and a heat source chamber of the heat exchanger 221 is communicated with the liquid return pipeline 232. The heater 222 is arranged in a pipeline between an outlet of the cold source chamber and an inlet of the heat source chamber.

It may be understood that based on that the temperature adjustment device 22 needs to simulate the temperature change working condition of the absorbent solution in a heating process of the desorption tower or a heat exchange process of the rich solution and the lean solution, the heater 222 and the heat exchanger 221 may be provided in the temperature adjustment device 22, the unheated absorbent solution flowing out of the carbon capture unit 1 is introduced into the cold source chamber of the heat exchanger 221, and the absorbent solution heated by the heater 222 is introduced into the heat source chamber of the heat exchanger 221, so that the two solutions may exchange heat in the heat exchanger 221, thereby simulating a temperature change condition of the absorbent solution in the heat exchange process of the lean solution and the rich solution. At the same time, the heater 222 may heat the introduced absorbent solution, thereby simulating a temperature change condition of the absorbent solution in the heating process of the desorption tower.

In some embodiments, as shown in FIG. 1, the temperature adjustment device 22 further comprises a cooler 223, the cooler 223 is arranged in an outlet pipeline of the heat source chamber to cool the high temperature absorbent solution flowing out of the heat source chamber.

It may be understood that based on that the high temperature absorbent solution needs to be cooled when returning to the absorbent tower, the cooler 223 may be added to the temperature adjustment device 22 of the above embodiment, and thus the cooler 223 may reduce the temperature of the absorbent solution.

In some embodiments, as shown in FIG. 1, the heater 222 is an electric heater or a heat pipe heater.

It may be understood that the heater 222 is the only heat source in the temperature adjustment device 22, and in case of a constant heat exchange efficiency of the heat exchanger 221, the heating temperature of the absorbent solution by the heater 222 directly affects whether the temperature change of the absorbent solution in this simulated working condition may be consistent with the actual carbon capture process, and the electric heater or heat pipe heater may realize the precise control of the heating temperature of the absorbent solution, ensuring to meet the experimental requirements. In some embodiments, the heat pipe heater is an oil bath heater.

In some embodiments, as shown in FIG. 1, the external circulation unit 2 further includes a defoamer 5, the driving device 21 is arranged between the liquid outlet of the carbon capture unit 1 and the temperature adjustment device 22, and the defoamer 5 is arranged between the driving device 21 and the temperature adjustment device 22.

It may be understood that the absorbent solution will flow through the defoamer 5 after being drawn out by the driving device 21 and then flow into the temperature adjustment device 22, and thus the defoamer 5 may remove bubbles mixed in the absorbent solution to avoid affecting the experimental effect.

In some embodiments, as shown in FIG. 1, the external circulation unit 2 further includes a metering pump 6, and the metering pump 6 is arranged between the defoamer 5 and the temperature adjustment device 22.

It may be understood that the metering pump 6 may adjust the flow rate of the absorbent solution input into the temperature adjustment device 22, so as to ensure that the absorbent solution may be uniformly heated or cooled in the temperature adjustment device 22, thus enhancing the reliability of the experimental results.

In some embodiments, as shown in FIG. 1, the external circulation unit 2 further includes a back pressure valve 4, and the back pressure valve 4 is arranged in the external circulation pipeline 23.

It may be understood that the back pressure valve 4 may prevent return of the absorbent solution and also maintain the stability of the flow rate of the absorbent solution, thus ensuring that the absorbent performance testing experiment may be carried out normally.

In some embodiments, the back pressure valve 4 is arranged between the cooler 223 and the carbon capture unit 1.

In some embodiments, the carbon capture absorbent performance testing system further includes a temperature detection assembly 3 (not shown), the temperature detection assembly 3 may be configured to detect temperatures at the liquid inlet and liquid outlet of the carbon capture unit 1, and temperatures at an inlet and an outlet of each element of the external circulation unit 2.

It may be understood that the temperature detection assembly 3 may dynamically reflect the temperature of the absorbent solution in each circulating element, and thus may be used to detect whether the absorbent solution achieves the desired temperature regulation effect. In case of a slip-up, the relevant experiment may be shut down in time, and the experiment may be continued after the slip-up is solved, thereby ensuring the accuracy of the experimental results.

In some embodiments, the temperature detection assembly 3 includes a plurality of thermometers, the plurality of thermometers may be correspondingly arranged at respective temperature measurement points. The plurality of thermometers are K-type stainless steel thermocouples.

It may be understood that the K-type stainless steel thermocouple may quickly and accurately measure the temperature and display the temperature measurement results using an electric meter, thereby facilitating the timely detection of temperature anomalies by the experimenter.

In the descriptions of the present disclosure, it is to be understood that the terms "central", "longitudinal", "transverse", "length", "width", "thickness", "up", "down", "front", "rear", "left", "right", "vertical", "horizontal", "top", "bottom", "inner", "outer", "clockwise", "counterclockwise", "axial", "radial", "circumferential", etc. indicate orientations or positional relationships based on those shown in drawings, are only intended to facilitate descriptions of the present disclosure and to simplify descriptions, and are not intended to indicate or imply that the device or element referred to must have a particular orientation, and be constructed and operated in a particular orientation. Therefore, these terms cannot be understood as limitations of the present disclosure.

Moreover, the terms "first" and "second" are used for descriptive purposes only and cannot be understood as indicating or implying relative importance or implicitly specifying the number of technical features indicated. Thus, a feature defined with the term "first" or "second" may expressly or impliedly include at least one such feature. In the description of the present disclosure, "a plurality of" means at least two, such as two, three, etc., unless expressly and specifically limited otherwise.

In the present disclosure, unless expressly specified or limited otherwise, the terms "mount", "connect", "couple", "fix", and the like should be understood broadly and may indicate, such as a fixed connection, a detachable connection, or a one-piece unit; a mechanical connection, an electrical connection, or a communication with each other; a direct connection, an indirect connection through an intermediate media, and intercommunication or interaction of two elements, unless expressly limited otherwise. For those skilled in the art, the specific meaning of the above terms in the present disclosure may be understood according to the specific situations.

In the present disclosure, unless specified or limited otherwise, the first feature being "above" or "below" the second feature may indicate a direct contact between the first feature and the second feature, or an indirect contact between the first feature and the second feature through an intermediate media. Furthermore, the first feature being "on", "above" and "over" the second feature may indicate that the first feature is directly above or obliquely above the second feature, or only indicate that the first feature is higher than the second feature in the horizontal direction. The first feature being "below", "under" and "underneath" the second feature may indicate that the first feature is directly below or obliquely below the second feature, or only indicate that the first feature is lower than the second feature in the horizontal direction.

In the present disclosure, the terms "an embodiment", "some embodiments", "an example", "a specific example", or "some examples" mean that the specific features, structures, materials, or characteristics described in connection with the embodiment or example are included in at least one embodiment or example of the present disclosure. In the specification, schematic expressions of the above terms do not necessarily refer to the same embodiments or examples. Furthermore, the specific features, structures, materials, or characteristics described may be combined in any one or more embodiments or examples in a suitable manner. Moreover, without contradicting each other, those skilled in the art may combine and associate different embodiments or examples and features of different embodiments or examples described in the specification.

Although the embodiments of the present disclosure have been shown and described above, it may be understood that the above embodiments are illustrative and are not to be understood as limitations of the present disclosure, and that those skilled in the art may make changes, modifications, substitutions, and variations of the above embodiments within the scope of the present disclosure.

## Claims

1. A carbon capture absorbent performance testing system, comprising:
a carbon capture unit configured to make carbon dioxide react with an absorbent solution to absorb the carbon dioxide;
an external circulation unit comprising an external circulation pipeline, a temperature adjustment device and a driving device, wherein an inlet of the external circulation pipeline is communicated with a liquid outlet of the carbon capture unit, an outlet of the external circulation pipeline is communicated with a liquid inlet of the carbon capture unit, the temperature adjustment device and the driving device are arranged in the external circulation pipeline, the temperature adjustment device is configured to adjust and control a temperature of the absorbent solution within the external circulation pipeline, and the driving device is configured to drive the absorbent solution to circularly flow between the external circulation pipeline and the carbon capture unit; and
a detection assembly configured to detect a carbon capture rate of the carbon capture unit.

2. The carbon capture absorbent performance testing system according to claim 1, wherein the detection assembly comprises a first detection member and a second detection member,
the first detection member is arranged at a gas inlet of the carbon capture unit to detect a content of carbon dioxide in incoming gas, and the second detection member is arranged at a gas outlet of the carbon capture unit to detect a content of carbon dioxide in outgoing gas.

3. The carbon capture absorbent performance testing system according to claim 1 or 2, wherein the temperature adjustment device comprises a heater and a heat exchanger, and the external circulation pipeline comprises a liquid intake pipeline in communication with the liquid outlet of the carbon capture unit and a liquid return pipeline in communication with the liquid inlet of the carbon capture device,
a cold source chamber of the heat exchanger is communicated with the liquid intake pipeline and a heat source chamber of the heat exchanger is communicated with the liquid return pipeline, and the heater is arranged in a pipeline between an outlet of the cold source chamber and an inlet of the heat source chamber.

4. The carbon capture absorbent performance testing system according to claim 3, wherein the temperature adjustment device further comprises a cooler, and the cooler is arranged in an outlet pipeline of the heat source chamber to cool a high temperature absorbent solution flowing out of the heat source chamber.

5. The carbon capture absorbent performance testing system according to claim 3 or 4, wherein the heater is an electric heater or a heat pipe heater.

6. The carbon capture absorbent performance testing system according to any one of claims 1 to 5, wherein the external circulation unit further comprises a defoamer,
the driving device is arranged between the liquid outlet of the carbon capture unit and the temperature adjustment device, and the defoamer is arranged between the driving device and the temperature adjustment device.

7. The carbon capture absorbent performance testing system according to claim 6, wherein the external circulation unit further comprises a metering pump, and the metering pump is arranged between the defoamer and the temperature adjustment device.

8. The carbon capture absorbent performance testing system according to any one of claims 1 to 7, wherein the external circulation unit further comprises a back pressure valve, and the back pressure valve is arranged in the external circulation pipeline.

9. The carbon capture absorbent performance testing system according to any one of claims 1 to 8, further comprising a temperature detection assembly, wherein the temperature detection assembly is configured to detect temperatures at the liquid inlet and the liquid outlet of the carbon capture unit, and temperatures at an inlet and an outlet of each element of the external circulation unit.

10. The carbon capture absorbent performance testing system according to claim 9, wherein the temperature detection assembly comprises a plurality of thermometers, the plurality of the thermometers are correspondingly arranged at respective temperature measurement points, and the plurality of thermometers are K-type stainless steel thermocouples.
